# EUROPÄISCHE PATENTANMELDUNG

(11) **EP 0 974 574 A1**
(43) Veröffentlichungstag der Anmeldung: **26.01.2000**
(21) Anmeldenummer: 99114155.7
(22) Anmeldetag: 21.07.1999
(51) Int. Cl.: C07C 51/43, C07C 57/07

(54) **Reinigung von Acrylsäure durch Kristallisation mittels Vakuumverdampfung**

(30) Priorität: 22.07.1998 DE 19832962
(71) Anmelder: BASF AKTIENGESELLSCHAFT, 67056 Ludwigshafen (DE)
(72) Erfinder: Eck, Bernd, 68519 Viernheim (DE); Heilek, Jörg, 69245 Bammental (DE); Schliephake, Volker, Dr., 67105 Schifferstadt (DE)
(74) Vertreter: Isenbruck, Günter, Dr.

(57) **Zusammenfassung**

In einem Verfahren zur Reinigung von Acrylsäure durch Kristallisation mittels Vakuumverdampfung wird eine Lösung, die Acrylsäure, Wasser und ggf. weitere Komponenten enthält, mittels Vakuumverdampfung unter Bildung einer Flüssigphase, bestehend aus einer Mutterlauge mit mehr als 10 Gew.-% Wasser und aus dem Kristallisat, und einer Dampfphase kristallisiert, die Dampfphase wird in einer Kondensationszone in ein flüssiges Material unter Ausbildung eines flüssigen Kondensatorgemisches eingebracht und das flüssige Kondensatorgemisch wird wenigstens teilweise in die Kondensationszone rückgeführt, wobei die Betriebsbedingungen in der Kondensationszone so eingestellt werden, daß kein Feststoff ausfällt.

## Beschreibung

Die vorliegende Erfindung betrifft ein Verfahren zur Reinigung von Acrylsäure durch Kristallisation mittels Vakuumverdampfung.

Kristallisationsverfahren zur Reinigung von Acrylsäure sind bekannt. Allgemein wird bei Kristallisationsverfahren nach der An der Erzeugung der zur Feststoffbildung nötigen Übersättigung zwischen Verfahren der Kühlungskristallisation und Verfahren der Verdampfungskristallisation unterschieden. Während bei der Kühlungskristallisation die Feststoffbildung durch Abkühlen der zu kristallisierenden Lösung erfolgt, wird bei der Verdampfungskristallisation die Löslichkeitsgrenze des zu kristallisierenden Feststoffs in der Lösung durch Verdampfung einer oder mehrerer Komponenten der Lösung überschritten (Lösungsmittelentzug). Erfolgt die Verdampfung im Vakuum (Vakuumverdampfung), kann durch das angelegte Vakuum die kristallisierende Lösung zusätzlich abgekühlt werden. Als Sonderfall einer Kristallisation mittels Vakuumverdampfung ist die Kristallisation mittels Siedekühlung zu sehen, bei der keine Wärmezuführ erfolgt und die Kristallisation damit als adiabate Vakuumverdampfungskristallisation ausgeführt wird. Der bei der Vakuumverdampfungskristallisation den Kristallisationsapparat verlassende Brüden (Dampf) wird üblicherweise in einer Kondensationsstufe kondensiert. Bei einer Siedekühlungskristallisation wird der kondensierte Brüden üblicherweise ganz oder teilweise in den Kristallisationsapparat zurückgeführt (Rückfluß des Kondensats in die Kristallisation). Die Siedekühlung als Vakuumverdampfungsverfahren ist daher zwischen dem reinen Kühlungsverfahren und dem reinen Verdampfungsverfahren (ohne Rückfluß des kondensierten Brüdens) anzusiedeln.

Gegenüber einem mit indirekter Kühlung über Wärmeübertrager arbeitenden Kühlungskristallisationsverfahren hat das Siedekühlungsverfahren den großen Vorteil einer direkten Kühlung der zu kristallisierenden Lösung (direkter Entzug der Verdampfungswärme aus der zu kristallisierenden Lösung). Dadurch werden Betriebsprobleme durch Verkrustungen, wie sie in den Wärmeübertragern einer indirekten Kühlung auftreten können, vermieden.

Aus der offengelegten japanischen Patentanmeldung JP 07 082 210-A ist ein Verfahren zur Reinigung von Acrylsäure durch Kristallisation mittels Siedekühlung bekannt. Bei diesem Verfahren wird von einer Rohsäure ausgegangen, die kein oder wenig Wasser enthält, und zu dieser Wasser zugegeben, um den Wassergehalt der bei der Kristallisation vorliegenden Mutterlauge auf einen Bereich zwischen 2 und 10 Gew.-% einzustellen. Teile des Acrylsäure/Wassergemisches werden verdampft, um unter Vakuum eine adiabatische Kühlung herbeizuführen, wodurch Acrylsäurekristalle ausfallen, die dann abgetrennt werden. Der bei der Verdampfung entstehende Dampf wird in einen Kondensator geleitet, wobei Acrylsäure auf der Oberfläche des Kondensators fließt, um eine Eisbildung zu verhindern, oder Wasser auf der Oberfläche des Kondensators fließt, um eine Ausfällung von Acrylsäurekristallen zu verhindern. Das Kondensat wird in die Kristallisation zurückgeführt.

Gegenüber diesem Stand der Technik lag der Erfindung die Aufgabe zugrunde, ein Verfahren zu schaffen, das die Reinigung von Acrylsäure aus einer Rohsäure mit einem höheren Wassergehalt erlaubt, wobei eine Feststoffbildung im Kondensator vermieden werden soll.

Es wird vorgeschlagen, dieses technische Problem dadurch zu lösen, indem man die Acrylsäure aus einer wasserhaltigen Lösung mittels Vakuumverdampfung unter Bildung einer Flüssigphase, bestehend aus einer acrylsäurehaltigen Lösung mit mehr als 10 Gew.-% Wasser (Mutterlauge) und dem Kristallisat, und einer Dampfphase auskristallisiert, die Dampfphase in einer Kondensationszone in ein flüssiges Material unter Ausbildung eines flüssigen Kondensationsgemisches einbringt und das Kondensationsgemisch wenigstens teilweise in die Kondensationszone zurückführt.

Somit betrifft die Erfindung ein Verfahren zur Reinigung von Acrylsäure durch Kristallisation mittels Vakuumverdampfung, wobei das Verfahren dadurch gekennzeichnet ist, daß eine Lösung, die Acrylsäure, Wasser und ggf. weitere Komponenten enthält, mittels Vakuumverdampfung unter Bildung einer Flüssigphase, bestehend aus einer Mutterlauge mit mehr als 10 Gew.-% Wasser und aus dem Kristallisat, und einer Dampfphase kristallisiert wird, die Dampfphase in einer Kondensationszone in ein flüssiges Material unter Ausbildung eines flüssigen Kondensationsgemisches oder Kondensatorgemisches eingebracht wird und das flüssige Kondensationsgemisch wenigstens teilweise in die Kondensationszone rückgeführt wird, wobei die Betriebsbedingungen in der Kondensationszone so eingestellt werden, daß darin kein Feststoff ausfällt.

Bevorzugte Ausführungsformen der Erfindung sind in den Unteransprüchen, der nachfolgenden Beschreibung mit den Figuren sowie dem Beispiel beschrieben. Vorliegend bezeichnen die Begriffe "Kondensatormischung" und "Kondensationsgemisch" das Gleiche. Die Begriffe "leichtsiedende Komponenten" und "mittelsiedende Komponenten" bezeichnen Komponenten, die einen niedrigeren Siedepunkt als Acrylsäure bzw. in etwa den gleichen Siedepunkt wie Acrylsäure haben.

Bei den Figuren zeigen:
- Fig. 1: ein Schema zur Durchführung eines bevorzugten Verfahrens gemäß der Erfindung, wobei externe Hilfsflüssigkeiten rückgeführtem Kondensationsgemisch zugeführt werden; und
- Fig. 2: ein Schema zur Durchführung eines bevorzugten Verfahrens gemäß der Erfindung, wobei externe und/oder interne Hilfsflüssigkeiten rückgeführtem Kondensationsgemisch zugeführt werden.

Geeignete Lösungen, aus denen die Acrylsäure erfindungsgemäß gewonnen werden kann, enthalten vorzugsweise 50 bis 97 Gew.-%, insbesondere 65 bis 95 Gew.-%, am meisten bevorzugt 70 bis 90 Acrylsäure und 3 bis 50 Gew.-%, insbesondere 5 bis 35 Gew.-%, am meisten bevorzugt 10 bis 30 Gew.-% Wasser, jeweils bezogen auf 100 Gew.-% Acrylsäure und Wasser. Zusätzlich zu der Acrylsäure und dem Wasser kann die zu kristallisierende Lösung weitere Komponenten, Verunreinigungen oder Nebenkomponenten in einer Menge von 0 bis 15 Gewichtsteilen, insbesondere von 0,3 bis 10 Gewichtsteilen, am meisten bevorzugt von 0,5 bis 5 Gewichtsteilen, jeweils bezogen auf 100 Gewichtsteile Acrylsäure und Wasser, enthalten. Bei den weiteren Komponenten/Nebenkomponenten handelt es sich insbesondere um bei der Acrylsäureherstellung durch katalytische Gasphasenoxidation von Propen/Acrolein anfallende Nebenkomponenten wie Essigsäure, Propionsäure, Formaldehyd, weitere Aldehyde, Maleinsäureanhydrid, leicht- und mittelsiedende Komponenten sowie nicht umgesetztes Propen oder Acrolein.

Die eingesetzten Kristallisationsverfahren und -apparate unterliegen grundsätzlich keiner Beschränkung. Es eignen sich alle Kristallisationsverfahren, bei denen eine Durchmischung der kristallisierenden Lösung durch Rühren oder Umwälzen erfolgt, sowohl bei kontinuierlicher als auch bei diskontinuierlicher Fahrweise. Als Apparate werden bevorzugt Rührkesselkristallisatoren und Forced Circulation-Kristallisatoren eingesetzt, aber auch Leitrohr- und Fließbettkristallisatoren können verwendet werden. Die Kristallisation wird mittels adiabater oder nicht-adiabater Vakuumverdampfung durchgeführt, d.h. die Verdampfung erfolgt im Vakuum entweder mit Wärmezufuhr (nicht-adiabatisch) oder ohne Wärmezufuhr (adiabatisch; Siedekühlung). Bei nicht-adiabater Vakuumverdampfung erfolgt die Wärmezufuhr über übliche Wärmetauscher, die keiner Beschränkung unterliegen. Zweckmäßigerweise werden Kristallisatoren mit außenliegenden Wärmetauschern oder mit Wandbeheizung verwendet. Vorzugsweise wird die Kristallisation im Vakuum bei 1 bis 8 mbar (absolut), insbesondere bei 2 bis 5 mbar (absolut), und bei einer Temperatur von -12 bis +5 °C, insbesondere von -5 bis +5 °C durchgeführt.

Die sich bei der Kristallisation bildende Dampfphase (Brüden) besteht größtenteils aus Wasser und Acrylsäure, kann aber auch Anteile an den weiteren Komponenten/Nebenkomponenten, wie insbesondere Essigsäure, Propionsäure, Aldehyde und leicht- und mittelsiedende Komponenten, enthalten. Die sich weiterhin bei der Kristallisation bildende Flüssigphase enthält das Acrylsäurekristallisat, das durch alle bekannten Verfahren der Fest-Flüssig-Trennung von der Mutterlauge abgetrennt werden kann. Vorteilhafterweise werden die Kristalle durch Filtrieren, Sedimentieren und/oder Zentrifugieren von der Mutterlauge abgetrennt, wobei das Kristallisat zusätzlich mit geeigneten Waschflüssigkeiten gewaschen werden kann.

Der Wassergehalt der Mutterlauge wird auf mehr als 10 Gew.-% Wasser, bezogen auf 100 Gew.-% Mutterlauge, eingestellt. Vorzugsweise liegt der für die Mutterlauge einzustellende Wassergehalt im Bereich zwischen 10 und 34 Gew.-%, bevorzugter zwischen 10 und 25 Gew.-%, am meisten bevorzugt zwischen 10 und 20 Gew.-% Wasser, jeweils bezogen auf 100 Gew.-% Mutterlauge. Die Mutterlauge enthält neben dem Wasser weiterhin Acrylsäure sowie gegebenenfalls weitere Komponenten/Nebenkomponenten. Die Einstellung des Wassergehaltes in der Mutterlauge kann der Fachmann leicht über den in der Kristallisation vorliegenden Druck und/oder über die in der Kristallisation zugeführte Wärmemenge vornehmen.

Das flüssige Material, in das die Dampfphase eingebracht oder kondensiert oder absorbiert wird, unterliegt an sich keiner besonderen Beschränkung. Es eignen sich alle flüssigen Materialien, die bei/nach Einbringung der Dampfphase zu einem flüssigen Kondensationsgemisch führen, über dem die zu kondensierenden oder absorbierenden Komponenten des Dampfes Partialdrücke aufweisen, die Meiner sind als die Partialdrücke dieser Komponenten über der kristallisierenden Lösung, und dessen Gefrierpunkt tiefer liegt als die Kondensations- oder Absorptionstemperatur. Vorliegend wird der Strom, der neben dem Brüden in die Kondensationszone eingebracht wird, als flüssiges Material bezeichnet, während der Strom, der die Kondensation verläßt, als Kondensationsmischung bezeichnet wird.

In einer bevorzugten Ausführungsform handelt es sich bei dem flüssigen Material um ein einphasiges flüssiges Gemisch, das 40 bis 75 Gew.-% Acrylsäure, insbesondere 50 bis 70 Gew.-% Acrylsäure, bevorzugterweise 62 bis 67 Gew.-% Acrylsäure, am meisten bevorzugt 65 bis 66 Gew.-% Acrylsäure und 25 bis 60 Gew.-% Wasser, insbesondere 30 bis 50 Gew.-% Wasser, bevorzugterweise 33 bis 38 Gew.-% Wasser, am meisten bevorzugt 34 bis 35 Gew.-% Wasser, jeweils bezogen auf 100 Gew.-% Acrylsäure und Wasser, enthält. Zweckmäßigerweise wird das flüssige Material unter Verwendung von zu kristallisierender Lösung, aus der Kristallisation kommender Mutterlauge, Flüssigphase, die Kristallisat enthält, oder einem Gemisch davon hergestellt.

Im kontinuierlichen Betrieb kann rückgeführtem Kondensationsgemisch eine Hilfsflüssigkeit zugeführt werden, mit der der Acrylsäuregehalt des dadurch erzeugten flüssigen Materials in Abhängigkeit von der Brüdenzusammensetzung und Brüdenmenge in einen Bereich von bevorzugt 50 bis 70 Gew.-%, insbesondere von 65 bis 66 Gew.-% Acrylsäure, jeweils bezogen auf 100 Gew.-% Acrylsäure und Wasser, gebracht wird. Vorzugsweise handelt es sich bei dieser Hilfsflüssigkeit um eine interne Hilfsflüssigkeit, d.h. eine flüssigkeit, die aus dem System stammt und bei der keine stoffsystemfremden Hilfsstoffe eingesetzt werden, und deren Acrylsäuregehalt wenigstens 65 Gew.-%, insbesondere 65 bis 95 Gew.-%, am meisten bevorzugt 70 bis 90 Gew.-%, jeweils bezogen auf 100 Gew.-% Acrylsäure und Wasser, beträgt. Besonders geeignet sind als interne Hilfsflüssigkeit zu kristallisierende Lösung, aus der Kristallisation kommende Mutterlauge, Flüssigphase, die Kristallisat enthält, oder ein Gemisch davon. Daneben kann auch eine externe Hilfsflüssigkeit verwendet werden. In einer bevorzugten Ausführungsform des Verfahrens wird die externe Hilfsflüssigkeit so gewählt, daß durch deren Einbringen in einem Anteil von wenigstens 5 Gew.-%, insbesondere von 5 bis 70 Gew-. %, am meisten bevorzugt von 5 bis 30 Gew-. %, jeweils bezogen auf das Kondensatorgemisch, die Gefriertemperatur des Kondensatorgemisches unter die Gefriertemperatur des Eutektikums Acrylsäure/Wasser (-12°C) abgesenkt wird. Bevorzugte Beispiele für externe Hilfsflüssigkeiten sind Essigsäure oder stark essigsäurehaltige Mischungen mit Acrylsäure und Wasser. Der Einsatz einer externen Hilfsflüssigkeit führt zu noch größeren Temperaturdifferenzen bei der Wärmeübertragung in der Kondensation und erlaubt damit den Einsatz kompakterer und wirtschaftlicherer Apparate. Jedoch kann er die gesonderte Aufarbeitung oder Entsorgung der Kondensatormischung erforderlich machen.

Die Einstellung der Betriebsbedingungen in der Kondensationszone, um ein Ausfällen von Feststoff darin zu verhindern, wird durch die Wahl des flüssigen Materials und durch die Gefriertemperatur des Kondensationsgemisches bestimmt. Der Fachmann kann im Rahmen dieser Parameter auf der Grundlage der vorliegenden Beschreibung leicht durch fachübliche Versuche geeignete Werte finden.

In einer bevorzugten Ausgestaltung der Erfindung liegt die Temperatur des flüssigen Kondensatorgemisches tiefer als die Sattdampftemperatur der Dampfphase.

Die Kondensationsverfahren und -apparate sowie die Erzeugung der für die Kondensation erforderlichen Stoff- und Wärmeaustauschflächen unterliegen keinerlei Beschränkung. Die zur Wärmeabfuhr erforderliche Übertragungsfläche kann in die Kondensationsstufe selbst als Oberflächenkondensator eingebracht sein oder im Umwälzkreislauf außerhalb des Kondensationsapparates liegen, der dann als Mischkondensator mit oder ohne Einbauten ausgeführt ist oder eine Kombination beider Varianten sein kann. Bevorzugt ist ein externer, einfacher und kostengünstiger Wärmeübertrager, wie z.B. ein Plattenwärmetauscher. Vorzugsweise erfolgt das Vermischen von Brüden und flüssigem Material durch Versprühen des flüssigen Materials und/oder durch Berieseln von Apparateflächen, Einbauten und/oder Füllkörpern des Kondensators mit dem flüssigen Material.

Erfindungsgemäß wird das Kondensatorgemisch wenigstens teilweise in die Kondensationszone rückgeführt. Vorzugsweise beträgt das Rückführverhältnis 10:1 bis 150:1, insbesondere 20:1 bis 150:1, am meisten bevorzugt 50:1 bis 150:1, jeweils bezogen auf kg/h (Massenstrom) rückgeführtes Kondensatorgemisch zu kg/h nicht-rückgeführtes Kondensatorgemisch. Nicht rückgeführtes Kondensatorgemisch wird aus dem Prozeß abgezogen oder, insbesondere wenn keine zusätzlichen Komponenten gegenüber den Komponenten in der zu kristallisierenden Lösung vorliegen, der Kristallisation rückgeführt.

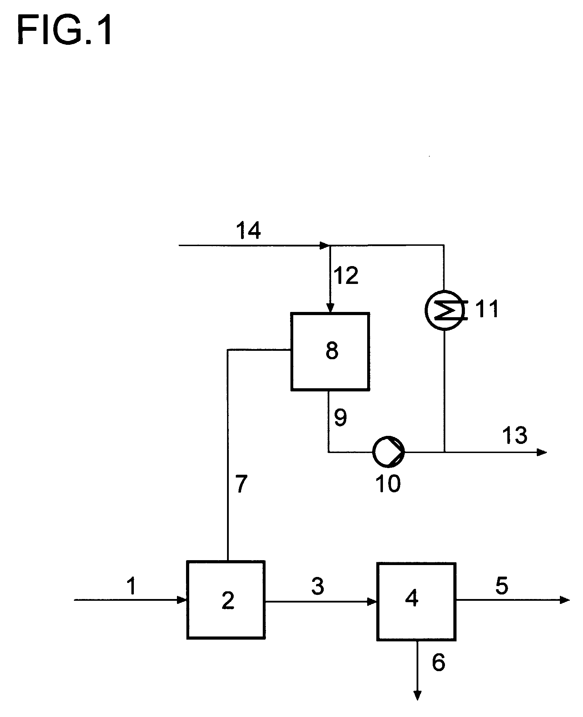

Fig. 1 zeigt eine bevorzugte Ausführungsform des erfindungsgemäßen Verfahrens. Eine Lösung 1, die die zu reinigende oder zu gewinnende Acrylsäure enthält, wird in die Kristallisation 2 gefahren, bei der der Wassergehalt der Mutterlauge über 10 Gew.-% gehalten wird. Über Leitung 3 wird die gebildete Kristallsuspension zur Fest-Flüssig-Trennung 4 geführt. Die abgetrennten Acrylsäurekristalle werden über Leitung 5 abgezogen, während die verbleibende Mutterlauge über die Leitung 6 abgezogen wird. Der bei der Kristallisation mittels Vakuumverdampfung gebildete Dampf (Brüden) wird über Leitung 7 in die Kondensationszone 8 geführt und dort mit flüssigem Material aus Leitung 12 vermischt. Über die Umlauf-/Rückführleitung 9, die Umwälzpumpe 10 und den Wärmeübertrager (extern) 11 wird rückgeführte Kondensatormischung zusammen mit externer Hilfsflüssigkeit aus Leitung 14 als flüssiges Material über Leitung 12 in die Kondensationszone 8 (rück)geführt. Nicht rückgeführte Kondensatormischung wird über die Leitung 13 ausgeschleust.

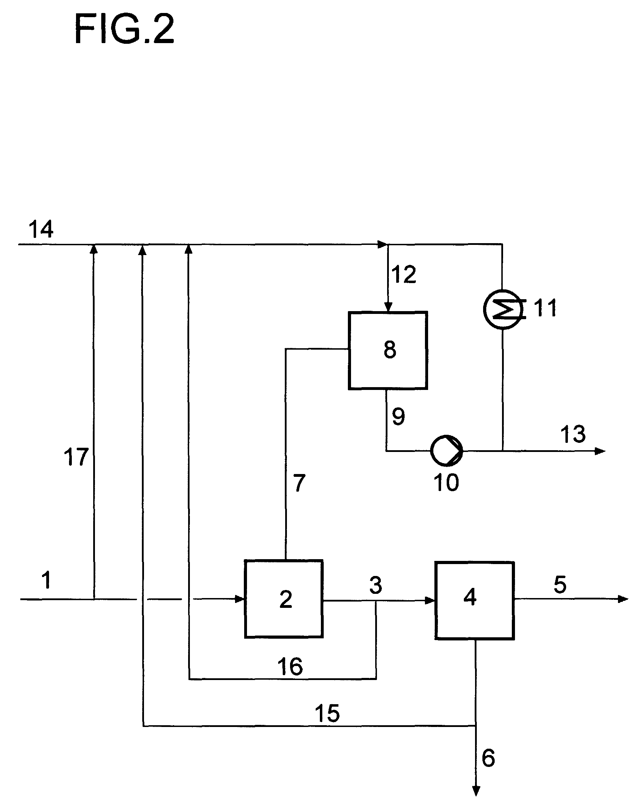

In Fig. 2 bezeichnen gleiche Bezugsziffern wie in Fig. 1 das Gleiche. Gegenüber

Fig. 1 weist Fig. 2 zusätzlich Leitungen zur Zuführung von Hilfsflüssigkeiten auf. Über Leitung 15 wird Mutterlauge, über Leitung 16 Kristallsuspension und über Leitung 17 zu kristallisierende Lösung, jeweils als Hilfsflüssigkeit der Leitung 14 zugeführt und über diese rückgeführter Kondensatormischung zugeführt.

In der JP 07 082 210-A wird Acrylsäure aus einer Rohsäure mit wenig oder keinem Wasser gewonnen, wobei der Wassergehalt der bei der Kristallisation vorliegenden Mutterlauge auf einen Bereich zwischen 2 und 10 Gew.-% eingestellt wird. Bei einem höheren Wassergehalt in der Mutterlauge wäre in JP 07 082 210-A Feststoffbildung im Kondensator zu erwarten. Demgegenüber erlaubt das erfindungsgemäße Verfahren, Acrylsäure aus einer Rohsäure mit einem erhöhten Wassergehalt zu gewinnen, ohne daß Feststoffbildung im Kondensator auftritt. Dies wird erreicht, indem bei einem Wassergehalt von mehr als 10 Gew.-% in der Mutterlauge gearbeitet wird und wenigstens ein Teil des Kondensationsgemisches zurückgeführt wird.

Die Erfindung wird anhand des folgenden Beispiels, das eine bevorzugte Ausführungsform der Erfindung darstellt, näher erläutert.

### BEISPIEL

Eine acrylsäurehaltige Lösung gemäß der in Tabelle 1 angegebenen Zusammensetzung wurde bei einem Massenstrom von 3340 g/h einem Kristallisationsapparat zugeführt.

**Tabelle 1**

| **Komponente** | **Konzentration in Gew.-%** |
|---|---|
| Wasser | 12,8 |
| Essigsäure | 1,0 |
| Acrylsäure | 85,4 |
| Maleinsäure 0,3 Acrolein 0,008 | |
| Propionsäure | 0,061 |
| Allylacrylat | 0,2 |
| Furfural | 0,2 |
| Phenothiazin (PTZ) | 0,013 |

Als Kristallisationsapparat wurde ein Rührbehälter mit einem Suspensionsvolumen von 5 l verwendet, der mit einem Wendelrührer ausgestattet war. Die bei der Kristallisation freiwerdende Wärme wurde über Teilverdampfung der Lösung abgeführt. Die Kristallisationstemperatur lag bei -1 °C, der Druck lag bei 4,5 mbar absolut. Der bei diesem Druck abgedampfte Brüden wurde in einem als Mischkondensator ausgebildeten Kondensator unter Verdüsung von flüssigem Material kondensiert. Zur Herstellung des flüssigen Materials zu Beginn wurde eine acrylsäurehaltige Lösung, die in ihrer Zusammensetzung identisch war mit der der Kristallisation zugeführten Lösung (Zusammensetzung gemäß Tabelle 1), verwendet, indem 100 Gewichtsteile dieser Lösung mit 30 Gewichtsteilen Wasser vermischt wurden. Der Druck im Kondensator entsprach nahezu dem Druck in der Kristallisation. Die in der Kondensationszone vorliegende Kondensatormischung hatte eine Temperatur von -9 bis -10 °C und folgende Zusammensetzung gemäß Tabelle 2.

**Tabelle 2**

| **Komponente** | **Konzentration in Gew.-%** |
|---|---|
| Wasser | 33,4 |
| Essigsäure | 0,04 |
| Acrylsäure | 64,8 |
| Maleinsäure | 0,2 |
| Acrolein | 0,067 |
| Propionsäure | 0,044 |
| Allylacrylat | 0,2 |
| Furfural | 0,1 |
| PTZ | 0,008 |

Von der den Kondensator verlassenden Kondensatormischung wurde ein Teilstrom von 375 g/h ausgeschleust. Der andere Teilstrom von etwa 26,5 kg/h wurde in den Kondensationsapparat rückgeführt, indem er zusammen mit einer acrylsäurehaltigen Lösung, die in ihrer Zusammensetzung identisch war mit der der Kristallisation zugeführten Lösung (Zusammensetzung gemäß Tabelle 1), als (interner) Hilfsflüssigkeit (vgl. Hilfsflüssigkeit aus Leitung 14 in Fig. 1) mit einem Massenstrom von 220 g/h vermischt wurde und dieses Gemisch als flüssiges Material in die Kondensationszone eingeführt wurde, das im Kondensator verdüst wurde. Die bei der Kristallisation erzeugte Suspension wurde auf einer 250 mm-Zentrifuge bei 1800 U/min und einer Schleuderzeit von 1 min in Kristalle und Mutterlauge aufgetrennt. Die Mutterlauge fiel in einer Menge von 2070 g/h an und hatte die Zusammensetzung gemäß folgender Tabelle 3.

**Tabelle 3**

| **Komponente** | **Konzentration in Gew.-%** |
|---|---|
| Wasser | 15,1 |
| Essigsäure | 1,4 |
| Acrylsäure | 82,3 |
| Maleinsäure | 0,5 |
| Acrolein | 0,0015 |
| Propionsäure | 0,084 |
| Allylacrylat | 0,21 |
| Furfural | 0,3 |
| PTZ | 0,02 |

Die Kristalle wurden nach Abtrennung der Mutterlauge auf der Zentrifuge mit geschmolzenem Kristallisat (300 g/h) bei 1800 U/min über 1 min gewaschen. Man erhielt eine Kristallisatmenge von 925 g/h. Das geschmolzene Kristallisat haue die Zusammensetzung gemäß folgender Tabelle 4.

**Tabelle 4**

| **Komponente** | **Konzentration in Gew.-%** |
|---|---|
| Wasser | 0,46 |
| Essigsäure | 0,18 |
| Acrylsäure | 99,3 |
| Maleinsäure | 0,019 |
| Acrolein | < 0,001 |
| Propionsäure | 0,018 |
| Allylacrylat | 0,01 |
| Furfural | 0,011 |
| PTZ | 0,0015 |

Wie aus dem Beispiel ersichtlich ist, ermöglicht das erfindungsgemäße Verfahren die Herstellung einer gereinigten Acrylsäure aus einer Rohsäure mit knapp 13 % Wassergehalt, wobei in der Kristallisation bei einem Wassergehalt in der Mutterlauge von 15,1 Gew.-% gearbeitet wird und eine Feststoffbildung im Kondensator vermieden wird.

## Patentansprüche

1. Verfahren zur Reinigung von Acrylsäure durch Kristallisation mittels Vakuumverdampfung, dadurch gekennzeichnet, daß eine Lösung, die Acrylsäure, Wasser und ggf. weitere Komponenten enthält, mittels Vakuumverdampfung unter Bildung einer Flüssigphase, bestehend aus einer Mutterlauge mit mehr als 10 Gew.-% Wasser und aus dem Kristallisat, und einer Dampfphase kristallisiert wird, die Dampfphase in einer Kondensationszone in ein flüssiges Material unter Ausbildung eines flüssigen Kondensationsgemisches eingebracht wird und das flüssige Kondensationsgemisch wenigstens teilweise in die Kondensationszone rückgeführt wird, wobei die Betriebsbedingungen in der Kondensationszone so eingestellt werden, daß darin kein Feststoff ausfällt.

2. Verfahren nach Anspruch 1, dadurch gekennzeichnet, daß die Lösung 50 bis 97 Gew.-% Acrylsäure und 3 bis 50 Gew.-% Wasser, jeweils bezogen auf 100 Gew.-% Acrylsäure und Wasser, enthält und weiterhin 0 bis 15 Gewichtsteile weitere Komponenten, bezogen auf 100 Gewichtsteile Acrylsäure und Wasser, enthält.

3. Verfahren nach Anspruch 1 oder 2, dadurch gekennzeichnet, daß die Kristallisation bei einem Druck von 1 bis 8 mbar (absolut) durchgeführt wird.

4. Verfahren nach einem der Ansprüche 1 bis 3, dadurch gekennzeichnet, daß der Wassergehalt in der Mutterlauge auf zwischen 10 und 34 Gew.-% eingestellt wird.

5. Verfahren nach einem der vorhergehenden Ansprüche, dadurch gekennzeichnet, daß als flüssiges Material ein einphasiges flüssiges Gemisch mit einem Acrylsäuregehalt von 50 bis 70 Gew.-% Acrylsäure bezogen auf 100 Gew.-% Acrylsäure und Wasser, eingesetzt wird.

6. Verfahren nach einem der vorhergehenden Ansprüche, dadurch gekennzeichnet, daß das flüssige Material aus rückgeführtem Kondensationsgemisch gebildet wird, dem eine interne Hilfsflüssigkeit mit einem Acrylsäuregehalt von wenigstens 65 Gew.-%, bezogen auf 100 Gew.-% Acrylsäure und Wasser, zugeführt wird, wobei diese Hilfsflüssigkeit aus zu kristallisierender Lösung, aus der Kristallisation kommender Mutterlauge, Flüssigphase, die Kristallisat enthält, oder einem Gemisch davon, ausgewählt wird.

7. Verfahren nach einem der vorhergehenden Ansprüche, dadurch gekennzeichnet, daß das flüssige Material aus rückgeführtem Kondensationsgemisch gebildet wird, dem eine externe Hilfsflüssigkeit zugeführt wird, deren Einbringen in einem Anteil von wenigstens 5 Gew.-%, bezogen auf das Kondensationsgemisch, die Gefriertemperatur des Kondensationsgemisches unter -12 °C absenkt.

8. Verfahren nach einem der vorhergehenden Ansprüche, dadurch gekennzeichnet, daß die Temperatur des flüssigen Kondensationsgemisches niedriger liegt als die Sattdampftemperatur der Dampfphase.

9. Verfahren nach einem der vorhergehenden Ansprüche, dadurch gekennzeichnet, daß das Rückführverhältnis für das Kondensationsgemisch in die Kondensationszone 10:1 bis 150:1, bezogen auf kg/h rückgeführtes Kondensationsgemisch zu kg/h nicht-rückgeführtes Kondensationsgemisch, beträgt.

10. Verfahren nach einem der vorhergehenden Ansprüche, dadurch gekennzeichnet, daß die Dampfphase mit dem flüssigen Material vermischt wird durch Versprühen des flüssigen Materials und/oder durch Berieseln von Apparateflächen, Einbauten und/oder Füllkörpern des Kondensators mit dem flüssigen Material.
